# EUROPEAN PATENT SPECIFICATION

(11) **EP 3 493 786 B1**
(45) Date of publication and mention of the grant of the patent: **26.05.2021**
(21) Application number: 16751438.9
(22) Date of filing: 03.08.2016
(51) Int. Cl.: A61K 8/31, A61K 8/34, A61Q 15/00, A61K 8/92

(54) **ACTIVE AGENT AND METHOD FOR REDUCING MALODOR**
WIRKSTOFF UM SCHLECHTEN GERUCH ZU VERRINGERN
ACTIF ET METHODE CORRESPONDANTE POUR REDUIRE LES MAUVAISES ODEURS

(43) Date of publication of application: 12.06.2019
(73) Proprietor: Colgate-Palmolive Company, New York, NY 10022 (US)
(72) Inventor: FAN, Aixing, Piscataway, New Jersey 08855 (US); SIMPSON, Edward, Monmouth Junction, New Jersey 08852 (US); DU-THUMM, Laurence D., Piscataway, New Jersey 08855 (US)
(74) Representative: Sonnenhauser, Thomas Martin
(86) International application number: PCT/US2016/045363
(87) International publication number: WO 2018/026364

(56) References cited:
- WO-A2-2004/022029
- US-A- 4 719 101
- US-A- 5 098 698
- US-A1- 2009 208 438

## Description

Body odor generally arises from the decomposition of mostly odorless perspiration by microorganisms on the skin. This is especially the case for body areas with large concentrations of sweat glands and odor-producing microorganisms, such as the armpits. The unpleasant smells or malodors associated with body odor are mainly caused by these degradation products. In particular, it is generally accepted that short and medium chain volatile fatty acids are among the primary molecules that cause axillary malodor.

Accordingly, there is a desire for active agents, compositions, and methods to suppress the odors associated with these short and medium chain volatile fatty acids.

### BRIEF SUMMARY

The foregoing and/or other aspects and utilities embodied in the present disclosure may be achieved by providing an active agent to reduce malodor associated with a short or medium chain volatile fatty acid, the active agent including an oleic fatty chain molecule having one or more hydroxide head groups wherein the oleic fatty chain molecule comprises shea butter glyceride.

In an embodiment, the oleic fatty chain molecule further includes one or more of a group consisting of an oleic alcohol, and an ethoxylated oleic alcohol.

In another embodiment, the one or more ethoxylated oleic alcohols include one or more polyethylene glycol ethers of oleic acid.

In another embodiment, the polyethylene glycol ethers of oleic acid include four or fewer ethylene oxide units.

In another embodiment, the polyethylene glycol ethers of oleic acid include Oleth-1, -2, - 3, or -4 ethoxylated oleic alcohols.

In another embodiment, the polyethylene glycol ethers of oleic acid include one or more of polyoxyethylene (2) oleyl ether and polyoxyethylene (3) oleyl ether.

The foregoing and/or other aspects and utilities embodied in the present disclosure may be achieved by providing a method of reducing a malodor associated with a volatile fatty acid (VFA), including topically applying to skin a composition as defined in the claims comprising an effective amount of an active agent to reduce the malodor associated with the VFA.

In another embodiment, the ethoxylated oleic alcohol includes four or fewer ethylene oxide units.

In another embodiment, the active agent includes Oleth-1, -2, -3, or -4 ethoxylated oleic alcohols.

The foregoing and/or other aspects and utilities embodied in the present disclosure may be achieved by providing an antiperspirant or deodorant composition as defined in the claims including an effective amount of active agent to reduce malodor associated with a short or medium chain volatile fatty acid.

According to the invention, the antiperspirant or deodorant composition includes from 0.5% to 10% active agent, based on a total weight of the antiperspirant or deodorant composition.

The foregoing and/or other aspects and utilities embodied in the present disclosure may be achieved by providing a composition as defined in the claims configured to reduce body malodor associated with short, which means C2-C5, or medium, which means C6-C11, chain volatile fatty acids (VFAs) including an effective amount of active agent to reduce malodor associated with a short or medium chain volatile fatty acid.

In another embodiment, the composition includes from about 2% to 5% active agent, based on a total weight of the composition.

### DETAILED DESCRIPTION

Reference will now be made in detail to the various embodiments in the present disclosure. The embodiments are described below to provide a more complete understanding of the components, processes, compositions, and apparatuses disclosed herein. Any examples given are intended to be illustrative, and not restrictive. However, it will be apparent to one of ordinary skill in the art that the invention may be practiced without these specific details. In other instances, well-known methods, procedures, and components have not been described in detail so as not to unnecessarily obscure aspects of the embodiments.

Throughout the specification and claims, the following terms take the meanings explicitly associated herein, unless the context clearly dictates otherwise. The phrases "in some embodiments" and "in an embodiment" as used herein do not necessarily refer to the same embodiment(s), though they may. Furthermore, the phrases "in another embodiment" and "in some other embodiments" as used herein do not necessarily refer to a different embodiment, although they may. As described below, various embodiments may be readily combined, without departing from the scope or spirit of the present disclosure.

As used herein, the term "or" is an inclusive operator, and is equivalent to the term "and/or," unless the context clearly dictates otherwise. The term "based on" is not exclusive and allows for being based on additional factors not described, unless the context clearly dictates otherwise. In the specification, the recitation of "at least one of A, B, and C," includes embodiments containing A, B, or C, multiple examples of A, B, or C, or combinations of A/B, A/C, B/C, A/B/B/ B/B/C, A/B/C, etc. In addition, throughout the specification, the meaning of "a," "an," and "the" include plural references. The meaning of "in" includes "in" and "on."

It will also be understood that, although the terms first, second, etc. may be used herein to describe various elements, these elements should not be limited by these terms. These terms are only used to distinguish one element from another. For example, a first object, component, or step could be termed a second object, component, or step, and, similarly, a second object, component, or step could be termed a first object, component, or step, without departing from the scope of the invention. The first object, component, or step, and the second object, component, or step, are both, objects, component, or steps, respectively, but they are not to be considered the same object, component, or step. It will be further understood that the terms "includes," "including," "comprises" and/or "comprising," when used in this specification, specify the presence of stated features, steps, operations, elements, and/or components, but do not preclude the presence or addition of one or more other features, steps, operations, elements, components, and/or groups thereof. Further, as used herein, the term "if' may be construed to mean "when" or "upon" or "in response to determining" or "in response to detecting," depending on the context.

All physical properties that are defined hereinafter are measured at 20° to 25° Celsius unless otherwise specified.

When referring to any numerical range of values herein, such ranges are understood to include each and every number and/or fraction between the stated range minimum and maximum, as well as the endpoints. For example, a range of 0.5-6% would expressly include all intermediate values of, for example, 0.6%, 0.7%, and 0.9%, all the way up to and including 5.95%, 5.97%, and 5.99%, among many others. The same applies to each other numerical property and/or elemental range set forth herein, unless the context clearly dictates otherwise.

Additionally, all numerical values take into account experimental error..

Unless otherwise specified, all percentages and amounts expressed herein and elsewhere in the specification should be understood to refer to percentages by weight. The amounts given are based on the active weight of the material.

With regard to procedures, methods, techniques, and workflows that are in accordance with some embodiments, some operations in the procedures, methods, techniques, and workflows disclosed herein may be combined and/or the order of some operations may be changed.

As described in the embodiments of the present disclosure, the inventors have surprisingly discovered new active agents with the ability to suppress the odors associated with short (C2-C5) and medium (C6-C11) chain volatile fatty acids (VFAs).

For example, although not intended to be bound by the theory, in one embodiment the new active agents form a complex with the VFAs to suppress the odors associated with these VFAs. In other embodiments, the new active agents may bind with the VFAs to suppress their odors.

The present invention thus refers to an antiperspirant or deodorant composition comprising an active agent to reduce malodor associated with a short, C2-C5, or medium, C6-C11, chain volatile fatty acid, the active agent comprising: an oleic fatty chain molecule having one or more hydroxide head groups, wherein the oleic fatty chain molecule comprises a shea butter glyceride; and wherein the antiperspirant or deodorant composition comprises from 0.5% to 10% of the active agent, based on a total weight of the antiperspirant or deodorant composition. It further refers to a method of reducing a malodor associated with a short, C2-C5, or medium, C6-C11, chain volatile fatty acid (VFA), comprising: topically applying to skin a composition of claim 1 to reduce the malodor associated with the VFA, wherein the active agent comprises an oleic fatty chain molecule having one or more hydroxide head groups, wherein the oleic fatty chain molecule comprises a shea butter glyceride. It also refers to a composition configured to reduce body malodor associated with short, C2-C5, or medium, C6-C11, chain volatile fatty acids (VFAs) comprising an active agent comprising an oleic fatty chain molecule having one or more hydroxide head groups, wherein the oleic fatty chain molecule comprises a shea butter glyceride, and wherein the composition comprises from 0.5% to 10% of the active agent, based on a total weight of the composition.

In one embodiment, the active agent includes an oleic alcohol (oleyl alcohol). Examples of usable oleic alcohols include, but are not limited to 9-Octadecen-1 -ol. For example, the oleic alcohol may include JARCOL 95CG available commercially from Jarchem Industries, Inc., headquartered in Newark, NJ, USA. In other embodiments, the oleic alcohol may include one or more commercially available oleic alcohols, such as, AEC oleyl alcohol (A&E Connock), HD-Eutanol (BASF Corporation), and Lipocol O/95 (Lipo Chemicals, Inc.).

In another embodiment, the active agent includes ethoxylated oleic alcohols. For example, in one embodiment, the active agent includes one or more polyethylene glycol ethers of oleic acid.

In some embodiments, the polyethylene glycol ethers of oleic acid include four or fewer ethylene oxide units. For example, in one embodiment, the ethoxylated oleic alcohol is an Oleth-1, -2, -3, or -4 ethoxylated oleic alcohol. In some embodiments, the ethoxylated oleic alcohol is an Oleth-2 or -3 ethoxylated oleic alcohol. In another embodiment, the active agent does not include ethoxylated oleic acids with more than four ethylene oxide units. In one embodiment, the active agent does not include ethoxylated oleic acids with more than three ethylene oxide units.

Examples of usable ethoxylated oleic alcohols include, but are not limited to polyoxyethylene (2) oleyl ether available commercially as Brij® O2, and polyoxyethylene (3) oleyl ether available commercially as Brij® O3, from Croda International PLC, headquartered in Snaith, UK In other embodiments, the ethoxylated oleic alcohol may include one or more commercially available ethoxylated oleic alcohols, such as, Eumulgin O2 (BASF Corporation), Lipocol O-2 and Lipocol O-3 (Lipo Chemicals, Inc.), Ritoleth-2 (Rita Corporation), and Jeecol OA-2, Jeecol OA-3 and Jeecol OA-4 (Jeen International Corporation).

According to the invention, the active agent includes shea butter glycerides. In one embodiment, the shea butter glyceride is derived mainly from stearic acid and oleic acid, and may include shea butter monoglycerides, shea butter diglycerides, shea butter triglycerides, and combinations thereof.

Aspects of the present disclosure may be further understood by referring to the following comparative examples and evaluations. These examples and evaluations are illustrative, and are not intended to be limiting embodiments thereof.

Active agents for reducing malodor may be included in deodorant and/or antiperspirant compositions, such as roll-on, aerosol or spray, or stick deodorant and/or antiperspirant products. For example, an agent for reducing malodor may be included in a roll-on antiperspirant deodorant composition for reducing underarm odor or other bodily malodor. In general, roll-on antiperspirant deodorant compositions may be in a liquid or gel form to reduce sweat and malodor, and may be applied to skin using a roll-on applicator.

### Example 1

Table 1 illustrates three deodorant compositions for reducing malodor. Composition A is a control roll-on deodorant composition, and Compositions B includes active agents according to some embodiments of the present disclosure. The composition of Compositions A and B, and Comparative Composition C, was identical except that 3wt% demineralized water in Composition A was replaced with either shea butter glyceride (Composition B) or oleic alcohol (Comparative Composition C).

**Table 1**

| **Component** | **Role** | **Composition A (control) (wt%)** | **Composition B (wt%)** | **Comparative Composition C (wt%)** |
|---|---|---|---|---|
| Demineralized water | Vehicle | 60 | 57 | 57 |
| Steareth-20 | Emulsifier | 0.75-1.75 | | |
| Caprylyl glycol | Preservative | 0.1-0.5 | | |
| PPG15 stearyl ether | Emollient | 0.5-2.0 | | |
| Steareth-2 | Emulsifier | 0.5-4 | | |
| BHT | Preservative | q.s. | | |
| Hydrogenated soybean oil | Emollient | 1-5 | | |
| EDTA dehydrate | Preservative | q.s. | | |
| 50% ACH solution | Antiperspirant active agent | 25-40 | | |
| Oleic alcohol | Active Agent | 0 | 0 | 3 |
| Shea Butter glyceride | Active Agent | 0 | 3 | 0 |

As described above, the roll-on deodorant compositions of Table 1 may include an active agent to reduce malodor. In addition, the roll-on deodorant compositions may also include other deodorant or antiperspirant agents, and may further include other ingredients commonly found in cosmetic and malodor-reducing composition. However, these additional ingredients are not meant to be limiting, and embodiments in the present disclosure may include additional or different ingredients in addition to the active agent to reduce malodor. In addition, while the present disclosure describes the use of the active agent to reduce malodor in a roll-on deodorant composition, the present invention is not limited thereto and the active agent to reduce malodor may also be used with other type of antiperspirant or deodorant compositions, such as gels, sticks, aerosols, creams, etc.

In some embodiments, an antiperspirant or deodorant composition may include an effective amount of the active agent to reduce malodor. As used herein, an "effective amount" means an amount of active agent sufficient to reduce a malodor associated with short (C2-C5) and medium (C6-C11) chain volatile fatty acids (VFAs), such as IVA and 3MH2. A "safe and effective amount" means an effective amount sufficient to induce a positive effect (reduction in malodor odor intensity) but low enough to avoid serious side effects (e.g., undue toxicity or allergic reaction, i.e., to provide a reasonable benefit to risk ratio, within the scope of sound medical judgment, etc.).

The composition includes from 0.5% to 10% of active agent based on a total weight of the composition. In another embodiment, the composition may include from 1% to 10%, or from 1.5% to 10% of active agent. In some embodiments, the composition may include from about 2% to about 5% of active agent or from about 3% to about 4% active agent. In one embodiment, the composition includes about 3% active agent based on the total weight of the composition.

### Antiperspirant Active

In some embodiments, the antiperspirant or deodorant composition may include antiperspirant actives in addition to the active agent to reduce malodor. When the composition includes an antiperspirant active, any of the known antiperspirant active materials can be utilized in the composition. Antiperspirant actives include, but are not limited to, aluminum chlorhydrate, aluminum chloride, aluminum sesquichloro hydrate, aluminum-zirconium hydroxychlorides, complexes or adducts of the above-mentioned active ingredients with glycol, such as propylene glycol (for example, "Rehydrol" II from Reheis Chemical Co.), and combinations thereof. Known aluminum-zirconium salts in combination with neutral amino acids, such as glycine (e.g., aluminum-zirconium tetrachlorohydrex Gly) can also be used. Generally, any of the Category I active antiperspirant ingredients, listed in the Food and Drug Administration's Monograph on Antiperspirant Drug Products for overall-the-counter human use (Oct. 10, 1973) can be used.

In other embodiments, the antiperspirant active is an aluminum salt and/or an aluminum-zirconium salt, such as those described above, that are further stabilized by betaine and a calcium salt. More information about betaine and calcium salt stabilized antiperspirant salts can be found in U.S. Patent Application Publication No. 2006/0204463 to Tang et al.

In some embodiments, the antiperspirant active, such as those described above, is selected to have a low metal to chloride ratio. Examples of these antiperspirant actives can be found in U.S. Patent No. 6,375,937 to Chopra et al, and in U.S. Patent No. 7,311,898 to Tang et al.

In other embodiments, the type of salt of interest, an aluminum zirconium tetrasalt or octasalt free of glycine are used wherein aluminum zirconium salt is stabilized by Betaine and has a metal to chloride ratio of about 0.9: 1 to about 1.3: 1 (and in other embodiments of about 0.9: 1 to about 1.2: 1 or about 0.9: 1 to about 1.1 : 1). For the tetrasalt, the Al/Zr atomic ratio can be about 3.2: 1 to about 4.1: 1.0 and the Betaine: zirconium mole ratio can be about 0.2: 1 to about 3.0: 1 (or in other embodiments of about 0.4: 1 to about 1.5: 1). Another salt that can be used is an aluminum chloride salt buffered by Betaine, wherein the salt has a metal to chloride ratio of 0.9: 1 to 1.3: 1 (and in other embodiments of about 0.9: 1 to about 1.2: 1 or about 0.9: 1 to about 1.1 : 1). For the octasalt the Al/Zr atomic ratio is about 6.2: 1 to about 10.0: 1 and the Betaine:Zr mole ratio is about 0.2: 1 to about 3.0: 1 (or in other embodiments of about 0.4: 1 to about 1.5: 1). In one embodiment, in the case of a salt that contains zirconium, the Betaine is incorporated during the synthesis of the salt so as to maximize the stabilizing effect this ingredient has (especially on the zirconium species). Alternatively, it can be post added to a glycine-free salt along with additional active phase ingredients to form a Betaine stabilized active.

Examples of commercially available glycine-free low M:C1 ratio tetrasalts and octasalts include, but are not limited to, REZAL™ AZP 955 CPG and REZAL™ AZP 885 respectively (both from Reheis Chemical Company, Berkeley Heights, N.J.). A more detailed description of making such commercially available salts can be found for example, in U.S. Patent Nos. 7,074,394 and 6,960,338. Further examples of making these types of salt complexes are described in U.S. Patent Application Publication No. 2004/0198998 and U.S. Patent No. 7,105,691.

In some embodiments, in addition to the anti- irritation properties of Betaine, it has also been found that antiperspirant formulations preserve their fragrance stability upon ageing when the Al/Zr salt is used in association with Betaine.

Additionally, the antiperspirant active can be a calcium salt stabilized antiperspirant active. Examples of calcium salt stabilized antiperspirant actives can be found in U.S. Patent No. 7,704,531.

In addition, any new ingredient, not listed in the Monograph, such as aluminum nitratohydrate and its combination with zirconyl hydroxychlorides and nitrates, or aluminum-stannous chlorohydrates, can be incorporated as an antiperspirant active. Antiperspirant actives can include, but are not limited to, the following: astringent salt of aluminum, astringent salt of zirconium, aluminum bromohydrate, aluminum chlorohydrate, aluminum dichlorohydrate, aluminum sesquichlorohydrate, aluminum chlorohydrex PG, aluminum dichlorohydrex PG, aluminum sesquichlorohydrex PG, aluminum chlorohydrex PEG, aluminum dichlorohydrex PEG, aluminum sesquichlorohydrex PEG, aluminum chloride, aluminum sulfate, aluminum zirconium chlorohydrate, aluminum zirconium trichlorohydrate, aluminum zirconium tetrachlorohydrate, aluminum zirconium pentachlorohydrate, aluminum zirconium octachlorohydrate, aluminum zirconium tetrachlorohydrex propylene glycol, aluminum zirconium trichlorohydrex Gly, aluminum zirconium tetrachlorohydrex Gly, aluminum zirconium pentachlorohydrex Gly, aluminum zirconium octachlorohydrex Gly, buffered aluminum sulfate, potassium alum, sodium aluminum chlorohydroxy lactate. In one embodiment, the antiperspirant active is aluminum chlorhydrate. In another embodiment, the antiperspirant active is aluminum zirconium tetrachlorohydrex propylene glycol.

### Surfactants

In some embodiments, the antiperspirant or deodorant composition may include surfactants in addition to the active agent to reduce malodor. Any surfactant that can be used in antiperspirant and/or deodorant compositions can be included. Examples of the surfactant include, but are not limited to, nonionic surfactants, silicone surfactants, and combinations thereof.

Nonionic surfactants that can be used include, but are not limited to, (a) sorbitan esters and ethoxylated sorbitan esters (for example PEG-20 sorbitan isostearate, sorbitan monolaurate, polysorbate-20, polysorbate-40, polysorbate-60, polysorbate-80); (b) ethoxylates (for example, Ceteth-20, PEG-30 castor oil, PEG-40 hydrogenated castor oil, PEG-60 hydrogenated castor oil, Laureth-7, Isolaureth-6, Steareth-10, Steareth-20, Steareth-21, Steareth-100, Ceteareth-12, Oleth-5, Oleth-10); (c) ethoxylated adducts (for example, PEG-25 stearate, glyceryl stearate and PEG-100 stearate); (d) PEG esters (for example, PEG-8 oleate, PEG-8 laurate, PEG-8 dilaurate, PEG-12 dilaurate, PEG-80 diisostearate, PEG-40 stearate): (e) propoxylates (for example, PPG-10 butanediol, PPG-50 oleyl ether, PPG-2-ceteareth-9, PPG-3-deceth-3, PPG-5-ceteth-20); (f) ethoxylated modified triglycerides (for example, PEG-20 corn glycerides, PEG- 12 palm kernel glycerides); (g) alkylphenol aromatic ethoxylates (for example, dinonylphenol ethoxylate with 9 moles of EO, octylphenol ethoxylate with 20 moles of EO, octylphenol ethoxylate with 40 moles of EO); (h) block copolymers that are alkoxylated glycols having ethoxylated and propoxylated segments (for example, POLOXAMER™ 182 and 234, POLOXAMER™ 105 Benzoate, and MEROXAPOL™ 174); and combinations thereof. In one embodiment, the nonionic surfactant is selected so that it has an HLB (hydrophilic-lipophilic balance) value of 8-16 (more particularly 8-12).

In one embodiment, the nonionic surfactant is selected from ethoxylated non-ionic surfactants and propoxylated non-ionic surfactants. Examples of these include, but are not limited to Steareth 2, Steareth 20, and Steareth 21. In an oil in water composition embodiment, a combination of 2 surfactants, one having an HLB value of about 2 to about 8 (such as Steareth 2) and the other having an HLB of about 9 to about 18 (such as Steareth 20 and 21), can be used.

Examples of silicone surfactants can be found in U.S. Patent No. 6,485,716. Suitable silicone surfactants include silicone polyglucosides (for example, octyl dimethicone ethoxy glucoside) and silicone copolyols having an HLB value (hydrophilic lipophilic balance) ≤ 8. The HLB value may be measured in a variety of ways such as described in conventional references or found listed in tables of data recording such values. It is intended that any type of HLB measurement technique may be used.

The surfactant can be included in any desired amount. In one embodiment, the amount of surfactant is about 0.1 to 15% of the antiperspirant or deodorant composition. In another embodiment, the surfactant comprises from about 2% to 12% of the composition, from about 3% to 10%, or from about 2% to 5%. The amount in the composition may be based on the as-supplied material.

### Parti culates

In some embodiments, the antiperspirant or deodorant composition may include particulates in addition to the active agent to reduce malodor. For example, the composition may also contain particulates which include, but are not limited to, talc, mica, fragrance encapsulates, or hydrophobically modified starches, such as aluminum starch octenyl succinate (MACKADERM™ ASTRO-DRY™ from Mclntyre Group Ltd.). If the composition is in a liquid form and dispensed through a roll-on applicator, the average particle size of the suspended material is sized so that it can pass through the application to prevent the ball applicator from malfunctioning. Usually, the average particle size does not exceed 150 microns.

In one embodiment, the amount of particulates is from about 0.1% to 30% of the antiperspirant or deodorant composition, in another embodiment from about 1% to 20%, or from about 5% to 15%.

### Malodor Counteracting Agents

In some embodiments, the antiperspirant or deodorant composition may include malodor counteracting agents in addition to the active agent to reduce malodor. For example, in certain embodiments, the composition may also contain at least one malodor counteracting alpha, beta-unsaturated ester or mixtures of such materials. In certain embodiments, the level of malodor counteracting composition to deliver a perceivable odor control benefit when delivered from an antiperspirant and/or deodorant composition is from about 0.05 to about 0.45 weight % based on the entire composition. The alpha, beta-unsaturated ester malodor counteracting materials are incorporated within the oil phase of an antiperspirant composition. Example of these malodor counteracting components can be found in U.S. Patent No. 6,610,648 and U.S. Patent No. 6,495,097. For example, in some embodiments, the odor neutralizing alpha, beta unsaturated ester mixture demonstrates unexpected stability in antiperspirant compositions containing low metal: chloride (M:C1) ratio salts free of glycine.

Examples of the alpha, beta unsaturated ester can be found in WO2005/025523, which was filed in the United States as U.S. Application Publication No. 2007/0196308.

In one embodiment, the amount of malodor counteracting agent is from about 0.05 to 20% of the composition, in another embodiment from about 0.1% to 20% of the composition, in another embodiment 0.5% to 15%.

### Carriers

In some embodiments, the antiperspirant or deodorant composition may include a carrier suitable for application to the skin. Such carriers include, but are not limited to, volatile silicones, emollients, lipophilic carrier materials or any combination of two or more thereof. The amount of the carrier material can vary widely depending on the type(s) of carrier, therefore the carrier can be present in a quantity of from about 0.1% to 98% of the composition.

### Volatile silicones

In one embodiment, the composition also comprises at least one volatile silicone component. Volatile compounds in the context of the invention are compounds which volatilize at body temperature, typically having a flash point of 100° C or less. Suitable volatile silicones, which may be linear, branched or cyclic, are described in Todd et al. "Volatile Silicone Fluids for Cosmetics", Cosmetics and Toiletries, pp. 27-32 (1976). Silicones containing 3 to 7 and more particularly 4 to 6 silicon atoms are preferred for the purposes of the invention. Particularly preferred are cyclic polydimethylsiloxanes such as, for example, octamethyl cyclotetrasiloxane, decamethyl cyclopentasiloxane or dodecamethyl cyclohexasiloxane which are known as cyclomethicones. They are commercially obtainable from G.E. Silicones as Cyclomethicone D-4 and D-5, from Dow Corning Corp. as Dow Corning® 344, 345 and 244, 245, 246, from General Electric Co. as GE® 7207 and 7158. One embodiment of the linear volatile silicones are those containing 1 to 7 and more particularly 2 to 3 silicon atoms. In another embodiment, the emollient is a volatile silicone is cyclomethicone or trisiloxane.

In some embodiments, the volatile silicones, when present, are typically present in a quantity of from about 0.1% to 98% of the composition, more particularly in a quantity of from about 1% to 90%, more particularly from about 5% to 70 %, and more particularly in a quantity of from about 10% to 35%.

### Emollients

In some embodiments, the antiperspirant or deodorant composition may include emollient in addition to the active agent to reduce malodor. The composition may contain non-volatile emollients in any desired amount to achieve a desired emollient effect. Emollients are known in the art and are used to impart a soothing effect on the skin. Classes of non-volatile emollients include non-silicone and silicone emollients. Non-volatile, non-silicone emollients include C12-15 alkyl benzoate. Non-volatile silicone material can be a polyethersiloxane, polyalkyarylsiloxane or polyethersiloxane copolymer. An illustrative non-volatile silicone material is phenyl trimethicone. Non-limiting examples of emollients can be found in U.S. Patent No. 6,007,799. Examples include, but are not limited to, PPG- 14 butyl ether, PPG- 15 stearyl ether, PPG-3 myristyl ether, stearyl alcohol, stearic acid, glyceryl monoricinoleate, isobutyl palmitate, glyceryl monostearate, isocetyl stearate, sulphated tallow, oleyl alcohol, propylene glycol, isopropyl laurate, mink oil, sorbitan stearate, cetyl alcohol, hydrogenated castor oil, stearyl stearate, hydrogenated soy glycerides, isopropyl isostearate, hexyl laurate, dimethyl brassylate, decyl oleate, diisopropyl adipate, n-dibutyl sebacate, diisopropyl sebacate, 2-ethyl hexyl palmitate, isononyl isononanoate, isodecyl isononanoate, isotridecyl isononanoate, 2-ethyl hexyl palmitate, 2-ethyl hexyl stearate, Di-(2-ethyl hexyl)adipate), Di-(2-ethyl hexyl) succinate, isopropyl myristate, isopropyl palmitate, isopropyl stearate, octacosanol, butyl stearate, glyceryl monostearate, polyethylene glycols, oleic acid, triethylene glycol, lanolin, castor oil, acetylated lanolin alcohols, acetylated lanolin, petrolatum, isopropyl ester of lanolin, fatty acids, mineral oils, butyl myristate, isostearic acid, palmitic acid, PEG-23 oleyl ether, olelyl oleate, isopropyl linoleate, cetyl lactate, lauryl lactate, myristyl lactate, quaternised hydroxy alkyl, aminogluconate, vegetable oils, isodecyl oleate, isostearyl neopentanoate, myristyl myristate, oleyl ethoxy myristate, diglycol stearate, ethylene glycol monostearate, myristyl stearate, isopropyl lanolate, paraffin waxes, glycyrrhizic acid, alkyl benzoate, hydrocyethyl stearate amide, and hydrogenated polyisobutene.

In one embodiment, the emollient is selected from linear silicones, cyclic silicones, hydrocarbons, polyhydroxy alcohols having more than 3 carbon atoms, liquid or solid polyalkyleneglycol ethers containing a polypropylene glycol (PPG) moiety and terminating in an alkyl ether, and combinations thereof. In another embodiment, the emollient is a nonvolatile silicone, such as dimethiconol or a longer chain dimethicone.

In one embodiment, the amount of emollient is from about 0.1% to 30% of the composition, in another embodiment from about 1% to 25% or from about 1% to 15%.

### Lipophilic Carrier Material

In some embodiments, the antiperspirant or deodorant composition may include a lipophilic carrier material in addition to the active agent to reduce malodor. For example, the composition may contain a lipophilic carrier comprising fat(s), oil(s), wax(s) or a combination thereof. These lipophilic components have structuring properties and provide the composition with the required consistency and with a particularly pleasant skin feel.

Any fats and fat-like substances may be used as part or all of the lipophilic carrier. These include inter alia fats (triglycerides), mono- and diglycerides, fatty alcohols, fatty acids, esters and/or ethers of fatty alcohols and fatty acids and also fatty acid amides or mixtures of these substances.

Waxes are understood to be natural or synthetic materials with the following properties: they are solid or fragile and hard in consistency, coarsely to finely crystalline, transparent or opaque and melt above 30° C without decomposing. They are low in viscosity and non-stringing only slightly above their melting point and show highly temperature-dependent consistency and solubility. Waxes suitable for use in accordance with the present disclosure are, for example, natural vegetable waxes with a melting point of 30 to 150° C such as, for example, candelilla wax, carnauba wax, Japan wax, espartograss wax, cork wax, guaruma wax, rice oil wax, sugar cane wax, ouricury wax, montan wax, sunflower wax, fruit waxes, such as orange waxes, lemon waxes, grapefruit wax, bayberry wax, and animal waxes such as, for example, beeswax, shellac wax, spermaceti, wool wax and uropygial fat. Natural waxes usable in accordance with the invention also include the mineral waxes, such as ceresine and ozocerite for example, or the petrochemical waxes, for example petrolatum, paraffin waxes and microwaxes. Other suitable wax components are chemically modified waxes, more particularly the hard waxes such as, for example, montan ester waxes, sasol waxes and hydrogenated jojoba waxes. Synthetic waxes usable in accordance with the invention include, for example, wax-like polyalkylene waxes and polyethylene glycol waxes.

The wax component may also be selected from the group of esters of saturated and/or unsaturated, branched and/or unbranched alkanecarboxylic acids and saturated and/or unsaturated, branched and/or unbranched alcohols, from the group of esters of aromatic carboxylic acids, dicarboxylic acids, tricarboxylic acids and hydroxycarboxylic acids (for example 12-hydroxystearic acid) and saturated and/or unsaturated, branched and/or unbranched alcohols and also from the group of lactides of long-chain hydro xycarboxylic acids providing the wax component or all the wax components melt at 30 to 150° C. Wax components such as these include, for example, CI 6-40 alkyl stearates, C20-40 alkyl stearates (for example Kesterwachs® K82H), C20-40 dialkyl esters of dimer acids, C18-38 alkyl hydroxystearoyl stearates or C20-40 alkyl erucates. Other suitable wax components which may be used with advantage are C30-50 alkyl beeswax, tristearyl citrate, triisostearyl citrate, stearyl heptanoate, stearyl octanoate, trilauryl citrate, ethylene glycol dipalmitate, ethylene glycol distearate, ethylene glycol di(12-hydroxystearate), stearyl stearate, palmityl stearate, stearyl behenate, cetyl ester, cetearyl behenate and behenyl behenate. Silicone waxes may also be used.

Suitable oil components are those in which the solids are homogeneously dispersed. A combination of nonpolar and polar oil component is possible. The nonpolar liquid oil components which can make up most of the carrier material include silicone oils and hydrocarbons which may be linear, branched or cyclic. Suitable hydrocarbons are, for example, isohexadecane, isododecane, polydecene and mineral oils such as, for example, thickly liquid and thinly liquid paraffins.

Examples of lipophilic components are described in U.S. Pat. Nos. 7,976,829; 6,849,251, and U.S. Pat. App. No. 2011/0274637, e.g., fats, oils and waxes.

In some embodiments, the lipophilic carrier material can be present in a total quantity of from about 0.1% to 60% of the composition, in another embodiment from about 1% to 50%, in another embodiment from about 1% to 20% or from about 5% to 15%.

### Other Deodorant Actives

In addition to the active agent to reduce malodor, in some embodiments, the composition may also contain other deodorant actives. Any known deodorant active may be used. Examples of other deodorant active include, but are not limited to antimicrobial actives, alcohols, 2,4,4'-trichloro-2'-hydroxy diphenyl ether (Triclosan), octoxyglycerin (SENSIVA™ SC 50), benzethonium chloride, polyhexamethylene biguanides, triethylcitrate, 2-amino-2-methyl-1-propanol (AMP), cetyl-trimethylammomium bromide, cetyl pyridinium chloride, bactericides, and bacterio stats.

In one embodiment, the amount of other deodorant active is from about 0.1% to 30% of the composition, in another embodiment from about 0.1% to 15% or from about 0.1% to 10%.

### Other Ingredients

A variety of fragrances can be used in these antiperspirant or deodorant compositions if a scented product is desired. Fragrances can be used in an amount in the range of from about 0.5% to 2%, particularly from about 0.01% to 2.0%, and, for example, at a level of about 1%.

Conventional gelling agent(s) may also be incorporated into the compositions. For example, stearyl alcohol and dibenzylidene sorbitol. When present, gelling agents are typically present in an amount of from about 0.1% to 30%, in another embodiment from about 7% to 15%. Gel products may also be made using polymers, for example polyethylene glycol.

Some ingredients listed herein can provide more than one function to the composition. For example, certain emollients can act as lipophilic carrier material and a gelling agent at the same time.

The ability of the active agents to reduce malodor was evaluated using both machine and human evaluations. In one embodiment, VFAs representative of body malodor were used for the evaluations. For example, the active agents were evaluated on their ability to reduce or suppress the malodor associated with isovaleric acid (IVA) and trans-3-methyl-2-hexanoic acid (3MH2). IVA and 3MH2 are examples of commonly found short and medium chain fatty acids associated with axillary, foot, and body malodors.

### Example 2

In one embodiment, isovaleric acid (IVA) (also known as 3-methyl butanoic acid, and available commercially from Sigma-Aldrich, headquartered in St. Louis, MO) and trans-3-methyl-2-hexanoic acid (3M2H) (available commercially from Narchem Corp., headquartered in Chicago, IL), were used as malodor markers (i.e., used to represent body malodor) to evaluate the ability of the active agents to suppress malodor as follows: 0.05 gram of a roll-on deodorant composition of Table 1 was weighed onto the tip of a cotton swab. Then, 150 µl of 0.1875% IVA solution or 0.25% 3M2H solution was added onto the cotton tip loaded with the roll-on deodorant composition. The loaded cotton tip was then placed in a clear 40 ml vial and incubated in a 37 °C oven for 30 minutes, 2 hours, and 4 hours, and 24 hours. Odor intensity was evaluated initially (before incubation) and then at each subsequent time interval. No odor was detected at the 24 hour interval. Each evaluation was performed in triplicate.

In one embodiment, *in-vitro* evaluations of Example 2 were performed using an electronic volatile organic compound detector. In particular, a Z-Nose detector (Model 4300 vapor analyzer and Model 3500 sample injector kit, available commercially from Electronic Sensor Technology, Inc., headquartered in Newbury Park, CA) was used to measure the odor intensity of the loaded cotton tips of Example 2 at each of the time intervals.

Table 2 illustrates the odor intensity of cotton tips loaded with IVA and each of the deodorant compositions of Table 1.

**Table 2**

| Composition | Incubation Time | | | |
|---|---|---|---|---|
| | Initial Intensity (counts) | 30 minutes (counts) | 2 hours (counts) | 4 hours (counts) |
| Composition A (control) | 2112±7 | 1154±28 | 1007±3 | 0 |
| Composition B (3% shea butter glyceride) | 1506±5 | 921±2 | 0 | 0 |
| Comparative Composition C (3% oleic alcohol) | 1405±4 | 0 | 0 | 0 |

Table 3 illustrates the odor intensity of cotton tips loaded with 3M2H for each of the deodorant compositions of Table 1.

**Table 3**

| Composition | Incubation Time | | | |
|---|---|---|---|---|
| | Initial Intensity (counts) | 30 minutes (counts) | 2 hours (counts) | 4 hours (counts) |
| Composition A (control) | 897±3 | 890±4 | 0 | 0 |
| Composition B (3% shea butter glyceride) | 0 | 0 | 0 | 0 |
| Comparative Composition C (3% oleic alcohol) | 803±3 | 0 | 0 | 0 |

As illustrated in Tables 2 and 3, the odor intensity associated with IVA and 3M2H was significantly reduced by the active agents in Composition B and Comparative Composition C as compared to the control Composition A, especially at the longer time intervals.

In another embodiment, human evaluations of Example 2 were performed using a panel of 13 people. Two IVA samples and a control sample were created according to Example 2. (3M2H samples were not used in the human evaluations). Each panelist was presented with a 3% shea butter glyceride sample (Composition B) and a control sample (Composition A) in randomized order, and was asked to use their noses to smell and rate the intensity of the odor of each sample. The panelists were asked to classify the two samples either having the same odor intensity (e.g., parity) or as one sample having a reduced odor intensity compared to the other. The samples were presented initially (before incubation) and then after 2 hours and 4 hours of incubation. The same process was then repeated using a 3% oleic alcohol sample (Comparative Composition C) and a control sample (Composition A) in randomized order.

Table 4 illustrates the panel results for odor intensity of cotton tips loaded with IVA for Composition B (3% shea butter glyceride).

**Table 4**

| Composition B Odor Intensity | Incubation Time (37°C) | | |
|---|---|---|---|
| | Initial | 2 hrs. | 4 hrs. |
| Parity w/ Composition A | 9 | 3 | 1 |
| Odor reduced v. Composition A | 4 | 10 | 12 |
| Odor increased v. Composition A | 0 | 0 | 0 |

Table 5 illustrates the panel results for odor intensity of cotton tips loaded with IVA for Comparative Composition C (3% oleic alcohol).

**Table 5**

| Comparative Composition C Odor Intensity | Incubation Time (37°C) | | |
|---|---|---|---|
| | Initial | 2 hrs. | 4 hrs. |
| Parity w/ Composition A | 11 | 3 | 1 |
| Odor reduced v. Composition A | 2 | 9 | 10 |
| Odor increased v. Composition A | 0 | 1 | 2 |

As illustrated in Tables 4 and 5, a substantial majority of the human panel confirmed the abilities of Composition B and Comparative Composition C to reduce the odor intensity associated with IVA, especially after 2 and 4 hours of incubation.

Accordingly, as illustrated in Tables 2-5, the inventors have surprisingly discovered new active agents capable of reducing a malodor associated with short (C2-C5) and medium (C6-C11) chain volatile fatty acids (VFAs), such as IVA and 3MH2.

While Tables 1-5 describe embodiments using the active agent in roll-on compositions. The present invention is not limited thereto. Instead, the active agents according to embodiments of the present disclosure may be employed in a variety of compositions which may be applied to skin for the reduction or elimination of body malodors, including stick-type or solid-type deodorant compositions.

Examples of compositions comprising embodiments of the active agent include antiperspirants, deodorants, shampoos, conditioners, skin cleansers, detergents, hair conditioners, sunscreens, sun tan lotions, skin conditioners, etc. It is to be understood that this list is not exhaustive with regard to suitable products comprising active agents according to embodiments of this disclosure. In addition, deodorant compositions comprising embodiments of the active agent may also comprise other materials commonly found in malodor-reducing compositions, such as deodorant or antiperspirant compositions, for example, cosmetically acceptable vehicles; deodorant actives; perfumes; antiperspirant actives; skin benefit agents; colorants; water; humectants and other cosmetic adjuncts conventionally employed in such compositions. The use of such substances depends on the form of the composition which may be an aerosol, stick, roll-on, gel, lotion, cream, ointment, powder, suspension, or soap.

## Claims

1. An antiperspirant or deodorant composition comprising an active agent to reduce malodor associated with a short, C2-C5, or medium, C6-C11, chain volatile fatty acid, the active agent comprising:
an oleic fatty chain molecule having one or more hydroxide head groups,
wherein the oleic fatty chain molecule comprises a shea butter glyceride; and
wherein the antiperspirant or deodorant composition comprises from 0.5% to 10% of the active agent, based on a total weight of the antiperspirant or deodorant composition.

2. The antiperspirant or deodorant composition of claim 1, wherein the oleic fatty chain molecule further comprises one or more of a group consisting of an oleic alcohol, and an ethoxylated oleic alcohol.

3. The antiperspirant or deodorant composition of claim 2, wherein the oleic fatty chain molecule further comprises one or more oleic alcohols.

4. The antiperspirant or deodorant composition of claim 2, wherein the oleic fatty chain molecule further comprises one or more ethoxylated oleic alcohols.

5. The antiperspirant or deodorant composition of claim 4, wherein the one or more ethoxylated oleic alcohols comprises one or more polyethylene glycol ethers of oleic acid.

6. The antiperspirant or deodorant composition of claim 5, wherein the polyethylene glycol ethers of oleic acid comprise four or fewer ethylene oxide units.

7. The antiperspirant or deodorant composition of claim 6, wherein the polyethylene glycol ethers of oleic acid comprise Oleth-1, -2, -3, or -4 ethoxylated oleic alcohols.

8. The antiperspirant or deodorant composition of claim 6, wherein the polyethylene glycol ethers of oleic acid comprise one or more of polyoxyethylene (2) oleyl ether and polyoxyethylene (3) oleyl ether.

9. A method of reducing a malodor associated with a short, C2-C5, or medium, C6-C11, chain volatile fatty acid (VFA), comprising:
topically applying to skin a composition of claim 1 to reduce the malodor associated with the VFA, wherein the active agent comprises an oleic fatty chain molecule having one or more hydroxide head groups, wherein the oleic fatty chain molecule comprises a shea butter glyceride.

10. The method of claim 9, wherein the active agent further comprises one or more of a group consisting of an oleic alcohol, and an ethoxylated oleic alcohol.

11. The method of claim 10, wherein the ethoxylated oleic alcohol comprises four or fewer ethylene oxide units.

12. The method of claim 11, wherein the active agent further comprises Oleth-1, - 2, -3, or -4 ethoxylated oleic alcohols.

13. A composition configured to reduce body malodor associated with short, C2-C5, or medium, C6-C11, chain volatile fatty acids (VFAs) comprising an active agent comprising an oleic fatty chain molecule having one or more hydroxide head groups, wherein the oleic fatty chain molecule comprises a shea butter glyceride, and wherein the composition comprises from 0.5% to 10% of the active agent, based on a total weight of the composition.

14. The composition of claim 13, wherein the composition comprises from about 2% to 5% of the active agent, based on a total weight of the composition.

## Patentansprüche

1. Antitranspirant- oder Deodorant-Zusammensetzung, umfassend ein aktives Mittel um schlechten Geruch, der mit einer kurz-, C2-C5-, oder mittel-, C6-C11-, kettigen, flüchtigen Fettsäure assoziiert ist, zu verringern, wobei das aktive Mittel umfasst:
ein Ölsäure-Fett-Ketten-Molekül mit einer oder mehreren Hydroxid-Kopfgruppen,
wobei das Ölsäure-Fett-Ketten-Molekül ein Sheabutter-Glycerid umfasst; und
wobei die Antitranspirant- oder Deodorant-Zusammensetzung von 0,5 % bis 10 % des aktiven Mittels umfasst, bezogen auf ein Gesamtgewicht der Antitranspirant- oder Deodorant-Zusammensetzung.

2. Antitranspirant- oder Deodorant-Zusammensetzung nach Anspruch 1, wobei das Ölsäure-Fett-Ketten-Molekül weiterhin einen oder mehrere aus einer Gruppe bestehend aus einem Ölsäure-Alkohol und einem ethoxylierten Ölsäure-Alkohol umfasst.

3. Antitranspirant- oder Deodorant-Zusammensetzung nach Anspruch 2, wobei das Ölsäure-Fett-Ketten-Molekül weiterhin einen oder mehrere Ölsäure-Alkohole umfasst.

4. Antitranspirant- oder Deodorant-Zusammensetzung nach Anspruch 2, wobei das Ölsäure-Fett-Ketten-Molekül weiterhin einen oder mehrere ethoxylierte Ölsäure-Alkohole umfasst.

5. Antitranspirant- oder Deodorant-Zusammensetzung nach Anspruch 4, wobei der eine oder die mehreren ethoxylierten Ölsäure-Alkohole einen oder mehrere Polyethylenglykolether der Ölsäure umfassen.

6. Antitranspirant- oder Deodorant-Zusammensetzung nach Anspruch 5, wobei die Polyethylenglykolether der Ölsäure vier oder weniger Ethylenoxideinheiten umfassen.

7. Antitranspirant- oder Deodorant-Zusammensetzung nach Anspruch 6, wobei die Polyethylenglykolether der Ölsäure Oleth-1, -2, -3 oder -4-ethoxylierte Ölsäure-Alkohole umfassen.

8. Antitranspirant- oder Deodorant-Zusammensetzung nach Anspruch 6, wobei die Polyethylenglykolether der Ölsäure einen oder mehrere von Polyoxyethylen-(2)-oleylether und Polyoxyethylen-(3)-oleylether umfassen.

9. Verfahren zum Reduzieren eines schlechten Geruchs, der mit einer kurz-, C2-C5-, oder mittel-, C6-C11-, kettigen, flüchtigen Fettsäure (VFA) assoziiert ist, umfassend:
topisches Auftragen einer Zusammensetzung nach Anspruch 1 auf die Haut, um den mit der VFA assoziierten schlechten Geruch zu reduzieren, wobei das aktive Mittel ein Ölsäure-Fett-Ketten-Molekül mit einer oder mehreren Hydroxid-Kopfgruppen umfasst, wobei das Ölsäure-Fett-Ketten-Molekül ein Sheabutter-Glycerid umfasst.

10. Verfahren nach Anspruch 9, wobei das aktive Mittel weiterhin einen oder mehrere aus einer Gruppe bestehend aus einem Ölsäure-Alkohol und einem ethoxylierten Ölsäure-Alkohol umfasst.

11. Verfahren nach Anspruch 10, wobei der ethoxylierte Ölsäure-Alkohol vier oder weniger Ethylenoxideinheiten umfasst.

12. Verfahren nach Anspruch 11, wobei das aktive Mittel weiterhin Oleth-1, -2, -3 oder -4-ethoxylierte Ölsäure-Alkohole umfasst.

13. Zusammensetzung, die beschaffen ist, um schlechten Körpergeruch, der mit kurz-, C2-C5-, oder mittel-, C6-C11-kettigen, flüchtigen Fettsäuren (VFAs) assoziiert ist, zu reduzieren, umfassend ein aktives Mittel, das ein Ölsäure-Fett-Ketten-Molekül mit einer oder mehreren Hydroxid-Kopfgruppen umfasst, wobei das Ölsäure-Fett-Ketten-Molekül ein Sheabutterglycerid umfasst und wobei die Zusammensetzung 0,5 % bis 10 % des aktiven Mittels, bezogen auf das Gesamtgewicht der Zusammensetzung, umfasst.

14. Zusammensetzung nach Anspruch 13, wobei die Zusammensetzung von etwa 2 % bis 5 % des aktiven Mittels, bezogen auf ein Gesamtgewicht der Zusammensetzung, umfasst.

## Revendications

1. Composition anti-transpirante ou déodorante comprenant un principe actif pour réduire une mauvaise odeur associée à un acide gras volatil à chaîne courte en C2-C5, ou moyenne en C6-C11, le principe actif comprenant :
une molécule à chaîne grasse oléique ayant un ou plusieurs groupes de tête hydroxyde,
dans laquelle la molécule à chaîne grasse oléique comprend un glycéride de beurre de karité ; et
dans laquelle la composition anti-transpirante ou déodorante comprend de 0,5 % à 10 % du principe actif, par rapport au poids total de la composition anti-transpirante ou déodorante.

2. Composition anti-transpirante ou déodorante selon la revendication 1, dans laquelle la molécule à chaîne grasse oléique comprend en outre un ou plusieurs d'un groupe constitué d'un alcool oléique et d'un alcool oléique éthoxylé.

3. Composition anti-transpirante ou déodorante selon la revendication 2, dans laquelle la molécule à chaîne grasse oléique comprend en outre un ou plusieurs alcools oléiques.

4. Composition anti-transpirante ou déodorante selon la revendication 2, dans laquelle la molécule à chaîne grasse oléique comprend en outre un ou plusieurs alcools oléiques éthoxylés.

5. Composition anti-transpirante ou déodorante selon la revendication 4, dans laquelle le ou les alcools oléiques éthoxylés comprennent un ou plusieurs éthers de polyéthylène glycol d'acide oléique.

6. Composition anti-transpirante ou déodorante selon la revendication 5, dans laquelle les éthers de polyéthylène glycol d'acide oléique comprennent quatre unités d'oxyde d'éthylène ou moins.

7. Composition anti-transpirante ou déodorante selon la revendication 6, dans laquelle les éthers de polyéthylène glycol d'acide oléique comprennent des alcools oléiques Oleth-1, -2, -3 ou -4 éthoxylés.

8. Composition anti-transpirante ou déodorante selon la revendication 6, dans laquelle les éthers de polyéthylène glycol d'acide oléique comprennent un ou plusieurs parmi l'éther oléylique polyoxyéthyléné (2) et l'éther oléylique polyoxyéthyléné (3).

9. Procédé de réduction d'une mauvaise odeur associée à un acide gras volatil (VFA) à chaîne courte en C2-C5, ou moyenne en C6-11, comprenant :
l'application topique sur la peau d'une composition selon la revendication 1 pour réduire la mauvaise odeur associée au VFA, dans laquelle le principe actif comprend une molécule à chaîne grasse oléique ayant un ou plusieurs groupes de tête hydroxyde, dans laquelle la molécule à chaîne grasse oléique comprend un glycéride de beurre de karité.

10. Procédé selon la revendication 9, dans lequel le principe actif comprend en outre un ou plusieurs d'un groupe constitué d'un alcool oléique et d'un alcool oléique éthoxylé.

11. Procédé selon la revendication 10, dans lequel l'alcool oléique éthoxylé comprend quatre unités oxyde d'éthylène ou moins.

12. Procédé selon la revendication 11, dans lequel le principe actif comprend en outre des alcools oléiques Oleth-1, - 2, -3 ou -4 éthoxylés.

13. Composition conçue pour réduire la mauvaise odeur corporelle associée à des acides gras volatils (AGV) à chaîne courte en C2-C5, ou moyenne en C6-C11, comprenant un principe actif comprenant une molécule à chaîne grasse oléique ayant un ou plusieurs groupes de tête hydroxyde, dans laquelle la molécule à chaîne grasse oléique comprend un glycéride de beurre de karité, et dans laquelle la composition comprend de 0,5 % à 10 % du principe actif, par rapport au poids total de la composition.

14. Composition selon la revendication 13, dans laquelle la composition comprend d'environ 2 % à 5 % du principe actif, par rapport au poids total de la composition.
